Europäisches Patentamt

European Patent Office

Office européen des brevets

(19)

(11) Veröffentlichungsnummer : **0 594 873 B1**

(12)                      **EUROPÄISCHE PATENTSCHRIFT**

(45) Veröffentlichungstag der Patentschrift :
**24.05.95 Patentblatt 95/21**

(51) Int. Cl.$^6$ : **G01N 33/574,** G01N 33/68, G01N 33/53

(21) Anmeldenummer : **92117983.4**

(22) Anmeldetag : **21.10.92**

(54) **Verfahren zur quantitativen Bestimmung von krankheitsspezifischen Antigenen und Vorrichtung zur Durchführung des Verfahrens.**

(43) Veröffentlichungstag der Anmeldung :
**04.05.94 Patentblatt 94/18**

(45) Bekanntmachung des Hinweises auf die
Patenterteilung :
**24.05.95 Patentblatt 95/21**

(84) Benannte Vertragsstaaten :
**AT CH DE FR GB IT LI NL**

(56) Entgegenhaltungen :
**EP-A- 0 153 283**
**EP-A- 0 196 845**
**US-A- 4 711 839**
**JOURNAL OF UROLOGY, Band 144, Nr. 6,**
**Dezember 1990, Baltimore, MD (US); H.C.B.**
**GRAVES et al., Seiten 1516-1522**
**CLINICAL CHEMISTRY, Band 38, Nr. 5, Mai**
**1992, Winston, NC (US); H.C.B. GRAVES et al.,**
**Seiten 735-742**

(73) Patentinhaber : **Huland, Edith, Dr. Dr.**
**Barkenkoppel 8**
**D-22391 Hamburg (DE)**
Patentinhaber : **Huland, Hartwig, Prof. Dr.**
**Barkenkoppel 8**
**D-22391 Hamburg (DE)**

(72) Erfinder : **Huland, Edith, Dr. Dr.**
**Barkenkoppel 8**
**W-2000 Hamburg 65 (DE)**

(74) Vertreter : **Fleck, Thomas, Dr. Dipl.-Chem. et al**
**Raffay & Fleck, Patentanwälte,**
**Postfach 32 32 17**
**D-20117 Hamburg (DE)**

EP 0 594 873 B1

## Beschreibung

Die Erfindung betrifft ein Verfahren zum quantitativen Bestimmen von krankheitsspezifischen Antigenen, insbesondere prostataspezifischen Antigenen, nach dem Oberbegriff des Anspruches 1 sowie eine Vorrichtung zur Durchführung dieses Verfahrens.

Prostataspezifischen Antigen (PSA) ist ein gewebespezifisches Glykoprotein, das nur in der Prostatadrüse und ihren Sekreten gefunden werden kann. Wegen der Gewebsspezifität des PSA stellt es bekanntermaßen einen einzigartigen Marker für die Prostatafunktion dar. (**Hara M, Kimura H**: Two prostate-specific antigens, γ-seminoprotein and β-microseminoprotein. J Lab Clin Med 1989; 113: 541-48. **Graves HCB, Kamarei M, Stamey TA**: Identity of prostate specific antigen and the semen protein p30 purified by a rapid chromatography technique. J Urol 1990; 144: 1510-5. **Sensabaugh GF, Blake ET**: Seminal plasma protein p30: Simplified purification and evidence for identity with prostate specific antigen. J Urol 1990; 144: 1523-26.) Es ist dadurch ein besonders nützlicher Marker, um Patienten nach radikaler Prostatektomie daraufhin zu kontrollieren, ob sie ein Wiederauftreten ihres Prostatakrebses erleiden, da der operative Eingriff das gesamte Prostatagewebe entfernt haben sollte und damit auch die mögliche Quelle für Freisetzung von prostataspezifischem Antigen. Bei Patienten mit einem Prostatakrebs, der sich durch histologische Überprüfung nachgewiesenermaßen auf das Organ Prostata beschränkt, fallen die Werte des PSA nach radikaler Prostatektomie unter die Nachweisgrenzen der zur Zeit verfügbaren Immunoassays in 80-92% der Fälle. Ein Wiederanstieg der PSA-Werte ist der frühestverfügbare Indikator für ein Wiederauftreten des Krebses nach kompletter Prostatektomie (**Stamey TA, Yang N, Hay AR, McNeal JE, Freiha FS, Redwine E**: Prostate-specific antigen as a serum marker for adenocarcinoma of the prostate. N Engl J Med 1987; 317: 909-16. **Oesterling JE, Chan DW, Epstein JI, Kimball AW, Bruzek DJ, Rock RC, Brendler CB, Walsh PC**: Prostate specific antigen in the preoperative and postoperative evaluation of localized prostatic cancer treated with radical prostatectomy. J Urol 1988; 139; 766-72. **Oesterling JE**: Prostate specific antigen: A critical assessment of the most useful tumor marker for adenocarcinoma of the prostate. J Urol 1991; 145: 907-23. **Lightner DJ, Lange PH, Reddy PK, Moore L**: Prostate specific antigen and local recurrence after radical prostatectomy. J Urol 1990; 144: 921-26). Die zur Zeit verfüglichen Immunoassays für PSA können PSA-Serumwerte zwischen 0,3 und 50 ng/ml nachweisen. Die Sensitivität der zwei führenden kommerziell erhältlichen Immunoassays für PSA liegt bei einer minimalen analytischen Sensitivität von 0,6-0,8 ng/ml PSA. (**Graves HCB, Wehner N, Stamey TA**: Comparison of a polyclonal and monoclonal immunoassay for PSA: Need for an international antigen standard. J Urol 1990; 144: 1516-22.) Bei vollständiger Entfernung einer normalen Prostata, wie dies bei Cystoprostatektomien wegen anderer Ursachen als Prostatakrebs vorliegt, konnte bei Patienten ein postoperativer PSA-Wert von weniger als 0,3 ng/ml durch den Yang Pros-check [R] PSA (**Stamey TA, Kabalin JN, McNeal JE, Johnstone IM, Freiha F, Redwine EA, Yang N**: Prostate specific antigen in the diagnosis and treatment of adenocarcinoma of the prostate. II. Radical prostatectomy treated patients. J Urol 1989; 141: 1076-83.) und <0,4-0,6 ng/ml durch Hybritech Tandem-R PSA-Assay (**Lange PH, Ercole CJ, Lightner DJ, Fraley EE, Vessella R**: The value of serum prostate specific antigen determinations before and after radical prostatectomy. J Urol 1989; 141: 873-79. **Hudson, MA, Bahnson RR, Catalona WJ**: Clinical use of prostate specific antigen in patients with prostate cancer. J Urol 1989; 142: 111-17.) gefunden werden. Es wird daher zur Zeit intensiv daran gearbeitet, einen sensitiveren Immunoassay zu finden, um niedrigere PSA-Level nachweisen zu können, die außerordentlich hilfreich darin sein können, frühzeitige Rezidive des Prostatakrebses bei Patienten nach radikaler Prostatektomie zu erkennen. Diese ultrasensitiven Assays haben einen Arbeitsbereich von 0,1-1,2 ng/ml und verbessern damit die analytische Sensitivität um etwa das Dreifache. Mit solchen ultrasensitiven Assays gehen aber Schwierigkeiten einher; beispielsweise sind die PSA-Kalibratoren im niedrigen Bereich (< 0,5 ng/ml) nicht stabil, wenn sie bei 4° aufbewahrt werden, so daß eine Kalibrationskurve jeden Tag erneut dadurch hergestellt werden muß, daß ein höherer Standardkalibrator (z.B. 8 ng/ml PSA-Kalibrator) verdünnt wird. Die mehrtägigen Inkubationszeiten sind aufwendig und erfordern strenge Temperatur- und bakterielle Kontaminationskontrollen (**Graves HCB, Wehner N, Stamey TA**: An ultrasensitive radioimmunoassay for prostate specific antigen. 1992, Clin Chem in press.)

Der vorliegenden Erfindung liegt deshalb die Aufgabe zugrunde, das eingangs genannte Verfahren derart zu verbessern, daß es die geschilderten Nachteile sicher vermeidet, generell auf krankheitsspezifische Antigene anwendbar ist und darüber hinaus technisch einfach in allen Laboratorien praktikabel ist und sich zudem mit anderen Verfahren der Sensitivitätsverbesserung gut kombinieren läßt. Diese Aufgabe wird durch das im Anspruch 1 gekennzeichnete Verfahren gelöst. Das erfindungsgemäße Verfahren beruht prinzipiell darauf, daß Serumproben durch Gefriertrocknung insbesondere auf 1/5, 1/10 oder 1/20 ihres ursprünglichen Volumens eingeengt werden. Es wird das Volumen der Ausgangsprobe bestimmt und das Volumen nach Einengung. Dieser Faktor wird bei der anschließenden Konzentrationsberechnung berücksichtigt. Die derart eingeengte Probe, die in ihrem Wassergehalt erheblich vermindert ist, aber nach wie vor das Prostata-spezifische Antigen mengenmäßig in der gleichen Menge enthält wie die Ausgangsprobe, kann dann in gewohnter Weise analysiert oder insbesondere mit herkömmlichen, kommerziell erhältlichen Immunoassays gemessen werden, da durch die derart gewonnene Konzentrierung auch ursprünglich niedrige Konzentrationen nun wieder im Meßbereich erscheinen. Nachdem die Probe mit Standardverfahren wie bisher gemessen wird, kann dann rechnerisch die Konzentration der Probe auf die Ausgangsserummenge ermittelt werden. Weitere vorteilhafte Merkmale sind in den Unteransprüchen beschrieben. Von besonderer erfindungsgemäßer Bedeutung ist ebenfalls eine Vorrichtung zur Durchführung des Verfahrens, die gekennzeichnet ist durch die Kombination mindestens der folgenden handelsüblichen Geräte: Ein Gerät zur Ermittlung des Probengewichtes und/oder Volumens vor oder nach der Gefrierkonzentrierung und eines Rechners zur Ermittlung des daraus resultierenden Konzentrationsfaktors sowie zur Zurückrechnung des ermittelten Wertes auf das Ausgangsgewicht bzw. Volumen der Patientenprobe in Verbindung mit Geräten zur Durchführung der Gefrierkonzentrierung selbst und der anschließenden Analyse bzw. des immunologischen Assays. Durch Kombination dieser Geräte kann eine weitgehende Automatisierung des beschriebenen Verfahrens zur Sensitivitätsverbesserung erreicht werden.

Es wird, wie nachfolgend beschrieben, erfindungsgemäß methodisch vorgegangen: Erfindungsgemäße Probenmessungen erfolgen z.B. als Doppel- oder Dreifachbestimmungen. Z.B. werden 10 ml der zu untersuchenden Serumprobe sowie jeweils z.B. 10 ml der entsprechenden Kontrollen und Kalibrierungsstandards tiefgefroren und in einer Gefriertrocknungsanlage über mehrere Stunden behandelt, bis das Gewicht der Probe auf etwa 5 oder 10 oder 20% des ursprünglichen Volumens eingeengt ist. Die Volumen und/oder Gewichtsmessungen vor und nach Gefriertrocknung haben mit größter Genauigkeit zu erfolgen. Hierdurch wird der Faktor ermittelt, um den nachher der Meßwert korrigiert werden muß, um die Konzentration der Ausgangsmenge zu erhalten. Zur Erleichterung dieses Vorgangs kann auch eine komplette Gefriertrocknung oder fast komplette Gefriertrocknung stattfinden und ein anschließendes Wiederauflösen bis zu einem definierten Gesamtvolumen oder Gesamtgewicht' z.B. 1 ml oder 2 ml. Die Lösung des hochkonzentrierten Serumlyophilisats wird durch kurze Inkubation von 15-30 Minuten im 37°C warmen Wasserbad erleichtert. Es ist auf eine genaue Durchmischung der gesamten Serumbestandteile zu achten. Die auf diesem Wege gewonnene Probe kann dann in herkömmlichen Assaysystemen gemessen werden (Yang Pros-check[R] PSA , Hybritech Tandem-R PSA assay, IMx PSA-assay und andere). Eine derart durchgeführte Einengung führt zu einer Konzentrierung der Probe um den Faktor 5 bis 20 und damit auch zu einer entsprechenden Sensitivitätsverbesserung.

In Tabelle 1 ist ein Beispiel für eine erfindungsgemäße Probenmessung angeführt.

| Verdünnung | Meßwert (ng/ml) | Sollwert (nach Verdünnung) (ng/ml) | Nach Gefrierkonzentrierung 10 ml auf 2 ml (ng/ml) | Zurückgerechnete Konzentration auf 10 ml |
|---|---|---|---|---|
| unverdünnt | 1,23 1,13 1,21 | 1,2 (unverdünnt) | 5,90 5,97 5,87  $\bar{x} = 5,91$ | 1,18 ng/ml |
| 1:1 | 0,57 0,51 0,56 | 0,6 | 2,65 2,51 2,58  $\bar{x} = 2,58$ | 0,51 ng/ml |
| 1:10 | 0,02 0,00 0,00 | 0,06 | 0,21 0,17 0,19  $\bar{x} = 0,19$ | 0,04 ng/ml |
| "0" Serum (weibl. Serum, gepoolt) | 0,00 0,00 0,00 | 0 | 0,00 0,00 0,00 | 0 ng/ml |

Tabelle 1: In diesem Beispiel für eine Probenmessung wurden 55 ml Serum mit einem PSA-Wert von 1,2 ng/ml hergestellt und gemessen und in verschiedenen Verdünnungsstufen mit sog. Nullserum (hierbei handelt es sich um gepooltes weibliches Serum, das keinerlei PSA enthält) gemessen. In der Säule 1 sind die verschiedenen Verdünnungen des Serums angegeben, in der Säule 2 der Meßwert, der sich aus der Analyse ergibt, ohne daß eine erfindungsgemäße Vorbehandlung erfolgt, in der Säule 3 der Sollwert, der sich errechnet aus den Verdünnungen ergeben müßte, und in der Säule 4 der nach Gefrierkonzentrierung von 10 ml auf 2 ml mit dem entsprechenden Enzymimmunoassay gemessene Wert, in der Säule 5 die ermittelte Konzentration durch Zurückrechnung auf 10 ml. In dem angegebenen Beispiel wurde das kommerziell erhältliche und weit verbreitete Assay System der IMx PSA Immunoassay der Firma Abbott verwendet, ein sog. Microparticle Enzyme Immunoassay MEIA.

In dieser Tabelle zeigt sich sehr deutlich in der 4. Zeile, daß bei einer 1:10-Verdünnung des ursprünglichen Serums der herkömmliche Assay ohne vorbehandelte Patientenprobe lediglich 0 Werte anzeigt und damit auch in der Dreifachbestimmung versagt. Bei Einsatz der Gefrierkonzentrierung läßt sich jedoch noch ein sicherer Meßwert ermitteln, der bei Zurückrechnung der Konzentration einen verläßlichen positiven Befund ergibt. Das "0"-Serum (weibliches Serum ohne PSA-Gehalt) hingegen bleibt auch nach Probenvorbehandlung durch Gefrierkonzentrierung komplett negativ).

Die Sensitivität der PSA-Messung kann also um bisher nicht erreichte Faktoren erhöht werden und dies unter Gebrauch herkömmlicher Analyse oder Immunoassayverfahren, so daß ein vorzeitiger Nachweis von Krankheitsspezifischen Antigenen möglich wird. Die Probenvorbereitung läßt sich aber durchaus auch mit suprasensitiven Meßbereiche in bislang unerreichbare Sensitivitätsbereiche möglich ist.

Eine weitere Konzentrierung der Probe durch Gefriertrocknung ist prinzipiell möglich. Unter Umständen muß dann in einem Zwischenschritt ein nicht relevanter wesentlicher Feststoff im Serum abgetrennt werden, um eine zu ausgeprägte Zähflüssigkeit der Probe zu vermeiden. Dies könnte z.B. durch gezielte Entfernungen von Albumin erreicht werden.

4

Erfindungsgemäß wird also ein neues Verfahren zum quantitativen Nachweis Prostata-spezifischen Antigens durch herkömmliche Immunoassays geschaffen, das in unteren Konzentrationsbereichen zuverlässige Aussagen ermöglicht. Dieses Verfahren ist dadurch gekennzeichnet, daß eine Probe zunächst einer Gefrierkonzentrierung unterworfen wird und erst daran anschließend eine Analyse oder ein Standardassay durchgeführt wird und daß das daraus resultierende Meßergebnis auf das ursprüngliche Probenvolumen zurückgerechnet wird. Die zu untersuchenden patientenprobe weist insbesondere einen PSA-gehalt von < 2 ng/ml, oder auch < 1,2 ng/ml auf.

Zwar ist die Konzentrierung durch Gefriertrocknung nicht prinzipiell neu, jedoch werden Gefriertrocknungen regelhaft und häufig nur eingesetzt, um die Stabilität von Proteinen, Geweben oder anderen biologischen Produkten zu erhöhen und auf diese Weise die entsprechenden Reagenzien lagerbar und haltbar zu machen. Auf diese Weise werden in immunologischen Testverfahren Standard, Kontrollen oder Reagenzien behandelt. Ein anderes bekanntes Einsetzverfahren für die Gefriertrocknung ist der Wunsch nach einer Gewichts- oder Platzersparnis. Wenn Proben Wasser entzogen wird, ohne daß sie sonst in ihrer Zusammensetzung verändert werden, können sie leichter transportiert werden, und eine anschließende Rekonstitution mit Wasser ist problemlos möglich. Dieses Prinzip der Gefriertrocknung kann dadurch auch auf der Seite der Lagerung einen erheblichen Vorteil bieten (vgl. beispielsweise die US-A-4994375). Die Stabilität von Substanzen bei längerfristiger Lagerung kann durch die Gefriertrocknung im allgemeinen sehr gut erhalten werden, zumTeil besser, zumindest aber gleich gut wie bei tief gefrorener Lagerung, so daß solche Überlegungen zur Erhaltung der Stabilität ebenfalls ein wichtiges Einsatzgebiet für die Gefriertrocknung darstellen.

Neu und überraschenderweise trotz des umfangreichen Einsatzes von Gefriertrocknungsmaßnahmen bislang noch nicht durchgeführt ist aber der verfahrensgemäßeEinsatz einer solchen effektiven Konzentrierungsmaßnahme, um eine bessere Sensitivität in der Messung von z.B. Tumormarkern, d.h. krankheitsspezifischen Antigenen, zu erzielen. Bislang wurde zur Verbesserung der Sensitivität einer Bestimmungsme thode überwiegend an der Assaymethode manipuliert. Die Einengung von Proben, um dadurch die Konzentration des entsprechenden Markers zu erhöhen und die Probe dadurch aus dem Hintergrundsrauschen in den meßbaren Bereich zu überführen, ist bislang noch nicht durchgeführt und durch die Literatur auch nicht nahegelegt worden. Das erfindungsgemäße Verfahren kann durchaus auch auf andere Substanzen übertragen werden, die in geringer Konzentration in Probenvolumina vorkommen, um auf diese Weise andere Tumormarker hochsensitiv nachzuweisen oder andere Substanzen, die in Körperflüssigkeiten in sehr geringer Konzentration vorkommen. Meßbare Erhöhungen der Konzentration von Patientenproben ließen sich mit Hilfe des beanspruchten Verfahrens auch bei anderen krankheitsspezifischen Antigenen nachweisen, so z.B. für das Interleukin-2. Auch dies ist eine Substanz, die normalerweise nicht im Serum vorkommt, aber bei Erkrankungen erhöht sein kann, z.B. bei der Abstoßung einer transplantierten Fremdniere. Hierbei besteht ebenfalls die Möglichkeit, durch Verbesserung der Nachweissensitivität die Diagnose früher stellen und die Therapie früher einleiten zu können, was dem Patienten erheblich zugute kommt.

Es dürfte dem Fachmann unmittelbar einleuchten, daß das erfindungsgemäße Verfahren nicht nur für die geschilderten Fälle PSA und Interleukin-2 anwendbar ist, sondern sich generell auf alle krankheitsspezifischen Antigene anwenden läßt. Bei vielen Erkrankungen sind die entsprechenden krankheitsspezifischen Antigene nach einer Ersttherapie oder auch im frühen Stadium zunächst im nicht meßbaren Bereich, und man geht davon aus, daß der Patient gesund bzw. geheilt ist. Erst mit einem Fortschreiten der Erkrankung nehmen die krankheitsspezifischen Antigene mengenmäßig zu und können dann mit herkömmlichen Meßverfahren nachgewiesen werden, dies allerdings zeitlich verspätet. Das erfindungsgemäße Verfahren geht dahin, daß Antigene, die in sehr niedriger Konzentration und damit in herkömmlichen Assays in nicht oder kaum meßbarem Bereich vorliegen, in der Patientenprobe so konzentriert werden, daß sie in einen meßbaren Bereich für herkömmliche Meßverfahren gebracht werden. Dieser vorzeitige Nachweis von krankheitsspezifischen Antigenen kann den Arzt in die Lage versetzen, Therapien früher und damit effektiver durchzuführen.

Bei dem beanspruchten Verfahren wird ausschließlich die Probenseite behandelt. Auf diese Weise ist das Verfahren kombinierbar mit allen herkömmlichen Meßverfahren. Zudem ist der Zeitfaktor überschaubar, da er lediglich vom Zeitaufwand, den die Gefriertrocknung einnimmt, abhängt. In der vorliegenden Untersuchungen stört die hohe Konzentration anderer Serumbestandteile in der eingeengten Probe die herkömmliche Messung (in unserem Beispiel IMx PSA assay der Firma Abbott) nicht, und die Einengung führt überraschenderweise in der Tat zu einem einfachen und zuverlässigen Verfahren zur Sensitivitätsverbesserung. Diese Überlegung ist grundlegend neu und wurde insbesondere für den Einsatz bei PSA bisher noch nicht angewandt, obwohl gerade hier seit Jahren intensiv nach ultrasensitiven Detektionsverfahren gesucht wird.

**Patentansprüche**

1. Verfahren zum quantitativen Bestimmen von krankheitsspezifischen Antigenen in einer Patientenprobe mit vorgegebenem Volumen oder Gewicht durch Analyse, dadurch gekennzeichnet, daß die Patientenprobe zunächst einer Gefrierkonzentrierung unterworfen und erst daran anschliessend analysiert wird, und daß das daraus resultierende Meßergebnis auf das ursprüngliche Probenvolumen oder -gewicht zurückgerechnet wird, wodurch eine Sensitivitätsverbesserung erzielt wird.

2. Verfahren nach Anspruch 1, dadurch gekennzeichnet, daß es sich bei den krankheitsspezifischen Antigenen um prostataspezifische Antigene (PSA), nämlich gewebespezifisches Glykoprotein, handelt.

3. Verfahren nach Anspruch 1, dadurch gekennzeichnet, daß es sich bei den krankheitsspezifischen Antigenen um Antigene handelt, die in der Patientenprobe in nicht messbarer Konzentration vorliegen.

4. Verfahren nach Anspruch 1 und 2, dadurch gekennzeichnet, daß die zu untersuchende Patientenprobe einen PSA-Gehalt < als 2 ng/ml aufweist.

5. Verfahren nach Anspruch 3, dadurch gekennzeichnet, daß der PSA-Gehalt der zu untersuchenden Patientenprobe bei < als 1,2 ng/ml liegt.

6. Verfahren nach einem oder mehreren der vorstehenden Ansprüche, dadurch gekennzeichnet, daß die Analyse in Form eines immunologischen assays durchgeführt wird.

7. Verfahren nach einem oder mehreren der vorstehenden Ansprüche 1 bis 4, dadurch gekennzeichnet, daß die Gefrierkonzentrierung auf 5 bis 20 Volumen-% der ursprünglichen Patientenprobe durchgeführt wird.

8. Verfahren nach einem oder mehreren der vorstehenden Ansprüche 1 bis 5, dadurch gekennzeichnet, daß die Gefrierkonzentrierung unter vorheriger oder anschließender Eliminierung eines nicht-relevanten Feststoffes, insbesondere Humanalbumin im Serum, durchgeführt wird.

9. Vorrichtung zur Durchführung des Verfahrens nach einem oder mehreren der vorstehenden Ansprüche unter Automatisierung zur Sensitivitätsverbesserung, gekennzeichnet durch die Kombination mindestens der folgenden handelsüblichen Geräte: Ein Gerät zur Ermittlung des Probengewichtes und/oder Volumens vor und nach der Gefrierkonzentrierung und eines Rechners zur Ermittlung des daraus resultierenden Konzentrationsfaktors sowie zur Zurückrechnung des ermittelten Wertes auf das Ausgangsgewicht bzw. Volumen der Patientenprobe in Verbindung mit Geräten zur Durchführung der Gefrierkonzentration selbst und der anschließenden Analyse bzw. des immunologischen Assays.


**Claims**

1. A process for the quantitative determination of illness specific antigens in a patient sample of a predetermined volume or weight by analysis, characterized in that a patient sample first undergoes a freeze concentration and is analyzed, and then the therefrom resulting product is calculated back to the original sample volume or weight, whereby a sensitivity improvement is attained.

2. Process according to claim 1 characterized in that the antigen is an illness specific antigen is a prostate specific antigen (PSA), namely a tissue specific glycoprotein.

3. Process according to claim 1 characterized in that the antigen is an antigen which is present in a non-measurable concentration in the patient sample.

4. Process according to claims 1 and 2 characterized in that the patient sample to be analyzed has a lower PSA content than 2 ng/ml.

5. Process according to claim 3 characterized in that the PSA content of the patient sample to be analyzed is < 1.2 ng/ml.

6. Process according to one or more of the previous claims characterized in that the analysis conducted is in the form of an immunoassay.

7. Process according to one or more of the previous claims 1 to 5 characterized in that the freeze concentration is conducted until 5 to 20 % by volume of the original patient sample is attained.

8. Process according to one or more of the previous claims 1 to 6 characterized in that the freeze concentration is conducted before or after the elimination of non-relevant solid materials, in particular human albumen in serum.

9. Apparatus for conducting the process according to one or more of the previous claims by automation for sensitivity improvement characterized by a combination of at least the following commercial devices: An apparatus for the determination of sample weight and/or volume, before and after freeze concentration, and a calculator for the determination of the resulting concentration factors therefrom, as well as for back calculating the determined value of the extraction weight and volume of the sample from the patient, respectively, in conjunction with devices for conducting the freeze concentration itself and the final analysis of the immunological assays.

**Revendications**

1. Procédé de détermination quantitative d'antigènes spécifiques des affections sur un prélèvement d'un volume ou poids défini par analyse, caractérisé par le fait, que le prélèvement soit soumis, dans un premier temps, à une cryoconcentration, l'analyse n'étant effectuée qu'à cette issue, et que le résultat de mesure en découlant soit reporté sur le volume ou poids initial de l'échantillon, ce qui permet d'obtenir une augmentation de la sensibilité.

2. Procédé selon la revendication 1, caractérisé par le fait que les antigènes spécifiques de l'affection sont en fait des antigènes spécifiques de la prostate (ASP), c.-à-d. des glycoprotéines tissulaires.

3. Procédé selon la revendication 1, caractérisé par le fait que les antigènes spécifiques de l'affection sont en fait des antigènes présents dans le prélèvement dans des concentrations se situant hors des limites de dosage.

4. Procédé selon les revendications 1 et 2, caractérisé par le fait que le prélèvement à analyser présente un taux d'ASP < 2 ng/ml.

5. Procédé selon la revendication 3, caractérisé par le fait que le taux d'ASP dans le prélèvement à analyser soit < 1,2 ng/ml.

6. Procédé selon une ou plusieurs des revendications précitées, caractérisé par le fait que l'analyse se fait à l'aide d'un immuno-test.

7. Procédé selon une ou plusieurs des revendications 1 à 4 précitées, caractérisé par le fait que la cryoconcentration du prélèvement aboutit à 5 - 20 % du volume initial.

8. Procédé selon une ou plusieurs des revendications 1 à 5 précitées, caractérisé par le fait que la cryoconcentration n'est exécutée qu'après élimination préliminaire ou subséquente d'une matière solide sans importance, en particulier l'albumine humaine, du sérum.

9. Dispositif pour la mise en application du procédé selon une ou plusieurs des revendications précitées avec automatisation de l'augmentation de la sensibilité, caractérisé par l'association d'au moins les appareils commercialisés suivants: un appareil pour la détermination du poids et/ou du volume avant et après la cryoconcentration et un calculateur pour la détermination du coefficient de concentration en résultant, ainsi que pour le report de la valeur déterminée sur le poids ou le volume initial du prélèvement, en association avec des appareils pour l'exécution de la cryoconcentration même et de l'analyse subséquente, voire de l'immuno-test.